# EUROPEAN PATENT SPECIFICATION

(11) **EP 4 601 543 B1**
(45) Date of publication and mention of the grant of the patent: **11.03.2026**
(21) Application number: 23786246.1
(22) Date of filing: 07.10.2023
(51) Int. Cl.: A61B 5/256, G01L 5/04, G01L 5/06, A61B 5/00

(54) **SYSTEM AND METHOD FOR POSITIONING A SENSOR PATCH ON A USER**
SYSTEM UND VERFAHREN ZUR POSITIONIERUNG EINES SENSORPATCHES AUF EINEM BENUTZER
SYSTÈME ET PROCÉDÉ DE POSITIONNEMENT D'UN TIMBRE DE CAPTEUR SUR UN UTILISATEUR

(30) Priority: 14.10.2022 EP 22201507
(43) Date of publication of application: 20.08.2025
(73) Proprietor: Koninklijke Philips N.V., 5656 AG Eindhoven (NL)
(72) Inventor: TE VELDE, Mart Kornelis-Jan, 5656 AG Eindhoven (NL); SMOOR, Divera Lucretia, 5656 AG Eindhoven (NL)
(74) Representative: Philips Intellectual Property & Standards
(86) International application number: PCT/EP2023/077806
(87) International publication number: WO 2024/079007

(56) References cited:
- US-A1- 2013 104 288

## Description

### FIELD OF THE INVENTION

The invention relates to a system and a method for positioning a sensor patch on a user. More specifically, it relates to a system comprising a band, at least permitting the monitoring of the stretch applied by the band to the user.

### BACKGROUND OF THE INVENTION

US2003144596A1 relates to a strap structure suitable for use with a biological information sensing device for sensing biological information such as arterial pulses or the like, as well as to a biological information sensing device using the same or more particularly, a biological information sensing device of a type like an arterial pulse wave detector which is wrapped around the wrist or the like.

US2013104288A1 relates generally to medical devices and, more particularly, to medical sensors used for sensing physiological parameters of a patient.

Sensor patches comprising electrodes can be used for electrophysiological measurements, which involve measurements of voltage changes or electric current in biological tissues. These measurements can be used for monitoring biometric parameters of a subject or for diagnostic purposes, e.g., based on electrocardiography (ECG) or electroencephalography (EEG). Another example is for pregnancy monitoring based on electrical-cardiotocography (eCTG), e.g. determining fetal heart rate (FHR), maternal heart rate (MHR) and uterine activity (UA). Sensor patches may comprise wet, dry or semi dry electrodes. For sensor patches which are designed to be re-used, dry electrodes are preferred, as they can be cleaned and they do not require gels or adhesives, which may be depleted after one or more uses. Sensor patches having dry electrodes can be in direct contact with the skin or can be worn on top of the clothes worn by a user.

To ensure good contact of the electrodes with the user, a certain amount of pressing force is required to be applied to the sensor patch and a stretchable band that is worn by the user can be used to create such a pressing force. The pressing force that the band exerts to the sensor patch can be considered proportional to the stretch of the band.

It is important to know if the amount of pressing force is the desired one. In case the pressing force is too high, it may create discomfort to the user. If on the other hand the pressing force is too low, it may lead to a poor contact between the electrode and the skin/clothing contact, resulting to incorrect functioning of the sensor patch and unreliable measurements.

There is thus a need for an improved system comprising a band, being able to at least permit the monitoring of stretch of the band, which indicates an amount of pressing force applied to the sensor patch.

### SUMMARY OF THE INVENTION

It is, inter alia, an object of the invention to provide a system comprising a band and a corresponding method, for positioning a sensor patch on a user, at least permitting the monitoring of the stretch applied by the band to the user. Using the system according to the invention, the user is able to at least observe if the amount of stretch is a desired one, if the band is under-stretched or overstretched and according to certain embodiments to adjust the amount of stretch.

The invention is defined by the independent claims. Advantageous embodiments are defined in the dependent claims.

A first aspect of the invention provides a system for positioning a sensor patch on a user, the system comprising a band for creating a pressing force to the sensor patch when the band is stretched, the band comprising a stretchable part, and a first marker located on the stretchable part, and a non-stretchable element, having a second marker, wherein the non-stretchable element is a ribbon, mounted on the band with a connector or an elastic joint at one end and an elastic joint at the other end, the ribbon even when taut, permitting the stretching of the band, and wherein a relative position of the first and second marker indicates an amount of stretch of the stretchable part.

An advantage thereof is that the user by visual inspection of the system may observe whether the stretchable part of the band has a desired stretch. The desired stretch corresponds to the desired force, i.e., the force desired by design which is required to press the sensor patch against the user. If the amount of stretch is the desired one, then a desired pressing force is achieved, reducing discomfort of the user and ensuring proper contact of the electrode with the skin or the clothes of the user, so that the sensor patch may take electrophysiological measurements according to its specifications. If it is observed that the stretch is less or more than desired, the user or another person can take action to bring the stretch of the band to a desired amount, e.g. by changing to another band which has dimensions more suitable to the size of the user, or by adjusting the amount of stretch, e.g., by using a fastener.

In case that the sensor patch is separable from the band and is designed such that the band can slide over the sensor patch when the user changes a position or moves, an advantage of ensuring that the band is not overstretched, is that motion artifacts may be reduced. If the stretch of the band exceeds a desired amount, then the high pressing force may effectively fasten the sensor patch on the band. As a result, the sliding of the band over the sensor patch will be hindered, leading to the dislocation of the sensor patch with respect to the skin of the user and to the creation of motion artifacts.

The non-stretchable element is a ribbon, e.g., a strip or band of fabric, mounted on the band, the non-stretchable element even when taut, permitting the stretching of the band. An advantage thereof is that when the band is worn, the non-stretchable element does not restrict the stretching of the band, even in the occasion that the band is stretched more than the desired amount. Otherwise, the user could potentially tear and irreparably damage the band, e.g., while attempting to wear it or attempting to increase the stretch more than the desired amount. Thus, these features enable a robust system, being less likely to be accidentally damaged by improper use.

The fabric of the ribbon may be silk, cotton, polyester, nylon, or polypropylene. Such materials improve wear comfort. Furthermore, such materials can be folded, and the fold does not damage them, e.g., when folded they mainly undergo temporary deformation.

The advantage of the non-stretchable element being a ribbon is that it is bendable and foldable. As a result, the system comprising the band and the ribbon can be easily folded and stored when not in use, requiring minimum storage space.

In an embodiment, the ribbon is irreversibly mounted on the band. As a result, usability is improved as the user does not need to attach the ribbon on the band. Furthermore, reliability is improved, because there is no risk that the ribbon may be detached or loosened. This could result to an incorrect indication of the amount of stretch of the stretchable part.

Furthermore, the structure of the system, i.e., that the ribbon, is mounted on the band with a connector or an elastic joint at one end and an elastic joint at the other end, ensures the integrity of the ribbon, even after prolonged usage of the system, resulting to a robust system with increased longevity. This effect is achieved because the ribbon is mounted on the band, i.e., it is attached to the band, but it does not form a structural element of the band. Thus, in case the system is under tension, i.e., when it is used, the tension needed for pressing the sensor patch against the user will mainly be transferred by the band itself, not by the ribbon. Namely, the ribbon is configured to convey a first tension force, which is less than a second tension force conveyed by the band. The tension force developed in the ribbon depends on the elasticity and amount of stretch of the elastic joint or joints used for mounting the ribbon on the band. For example, the first tension force in the ribbon can be less than 10% and more preferably less than 1% of the second tension force in the belt.

In an embodiment, the stretchable part may comprise at least two first markers, which are symmetrical about an axis extending along a width of the band and the non-stretchable element is mounted on the band with elastic joints at both of its ends, wherein the non-stretchable element comprises at least two second markers, which are symmetrical about the same axis. An advantage thereof is that by having two or more first and second markers, the user is provided with extra markers to check the stretch of the band, resulting in improved usability of the system. For example, if one pair of first and second markers is out of sight of the user e.g., an obstacle is present or the body part where the band is worn may not permit easy inspection, at least another pair of first and second markers may be visible.

According to an embodiment, the system may comprise a fastener for adjusting an amount of stretch of the stretchable part. An advantage thereof is that the user is able to adjust the stretch of the band, in order to reach a desired stretch and thus a desired pressing force. In case the system does not comprise a fastener, the user is limited to observing the amount of stretch of the stretchable part of the band.

According to an embodiment, the band comprises a non-stretchable part, wherein the non-stretchable part is less stretchable than the stretchable part, and wherein at least one of the non-stretchable element and the fastener are mounted on the non-stretchable part. The material of the non-stretchable part, being more rigid, permits a more stable and more robust connection with the non-stretchable element and/or the fastener, compared to a similar connection with the stretchable part.

In a second aspect of the invention a method for positioning a sensor patch on a user is provided, the method comprising positioning the sensor part on the user, positioning on the user a system as described herein, and monitoring the amount of stretch of the stretchable part of the band indicated by the relative position of the first and second marker.

Additionally, in case the described system comprises a fastener, the method further enables adjusting the amount of stretch of the stretchable part of the band, by securing the fastener in a desired position on the band.

The advantages and technical effects as already described for the system also apply to the corresponding method.

These and other aspects of the invention will be apparent from and elucidated with reference to the embodiments described hereinafter.

### BRIEF DESCRIPTION OF THE DRAWINGS

Figs. 1a - 1d show an example of the system for positioning a sensor patch on a user according to the invention.
Figs. 2a - 2d show another example of the system for positioning a sensor patch on a user according to the invention.
Figs. 3a - 3b show an example of the system for positioning a sensor patch on a user according to the invention.
Fig. 4 shows a flowchart of a method for positioning a sensor patch on a user according to the invention.

### DESCRIPTION OF EMBODIMENTS

The invention will be described with reference to the Figures. The detailed description and specific examples, while indicating exemplary embodiments of the invention, are intended for purposes of illustration only and are not intended to limit the scope of the invention. These and other features, aspects, and advantages of the present invention will become better understood from the following description, appended claims, and accompanying drawings. The Figures are merely schematic and are not drawn to scale. It should also be understood that the same reference numerals are used throughout the Figures to indicate the same or similar parts.

The invention provides a system and a method for positioning a sensor patch on a user. The sensor patch may be used for electrophysiological measurements e.g., based on electrocardiography (ECG), electroencephalography (EEG) or electrical-cardiotocography (eCTG). The user's body part where the sensor patch should be positioned depends on the measurements to be taken. For example, a sensor patch monitoring ECG should preferably be placed at a chest area of the user, a sensor patch monitoring EEG should preferably be placed at the head of the user, and a sensor patch used for pregnancy monitoring should be placed at the belly of the user.

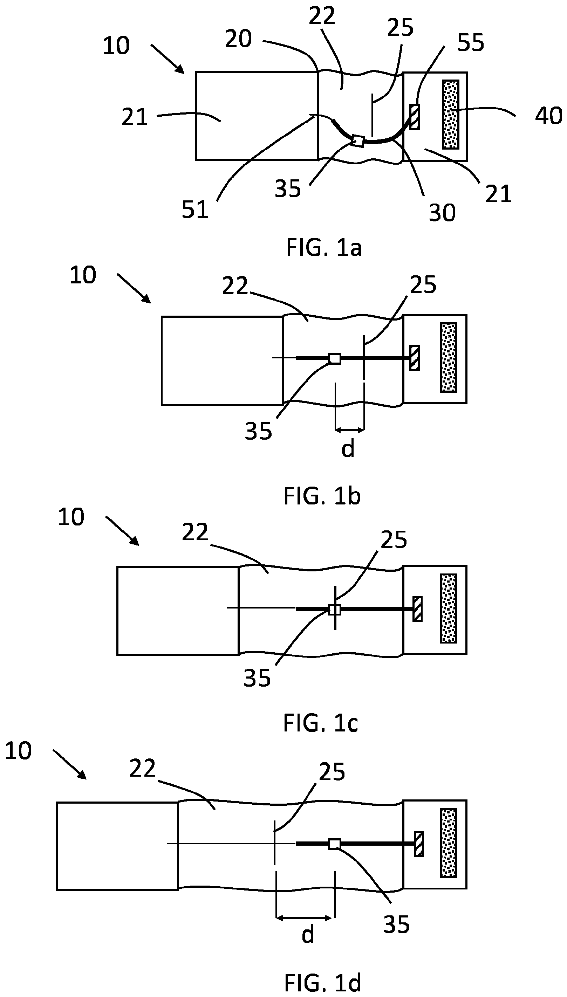

Figs 1a to 1d show a system 10 for positioning a sensor patch on a user. The system 10 comprises a band 20, the band having a stretchable part 22 wherein a first marker 25 is located. The system also comprises a non-stretchable element 30, having a second marker 35. **In** Figs 1a to 1d the non-stretchable element 30 is a ribbon. The non-stretchable element's 30 length does not substantially change under tensile forces, e.g., it increases less than 5%, preferably less than 2% and more preferably less than 1% of the length it has in untaut position.

The band 20, e.g., a belt, creates a pressing force to the sensor patch when the band 20 is stretched, thus, keeping the sensor patch positioned on the user. The sensor patch may comprise wet, semi-dry or dry electrodes.

The stretchable part 22 of the band 20 may be made of any suitable materials, e.g., stretch fabrics, having stretchable properties, e.g., nylon, elastane, neoprene. Preferably, the stretchable part 22 of the band 20 is made of materials appropriate for clothing and/or is washable. The stretchable part 22 may stretch in one direction only, i.e., either lengthwise or crosswise or in two directions, i.e., both lengthwise and crosswise. Preferably the stretchable part 22 at least partly recovers to its original position when the band 20 is not stretched. The stretchable material may be able to stretch more than 20%, preferably more than 40% and even more preferably in a range between 30% to 60% of its width and/or length in unstretched position, without losing its ability to at least partially recover to its unstretched position.

Each of the disclosed ranges regarding stretchability of the stretchable part 22 can be combined with each of the disclosed ranges regarding non-stretchability of the non-stretchable element 30.

In order to protect the stretchable material from stretching above a point that it may be damaged, the stretchable part 22 may comprise a protecting element, e.g., a ribbon. The protecting element may restrict the stretchable part 22 from stretching more than 100% to 120% of its width and/or length in unstretched position.

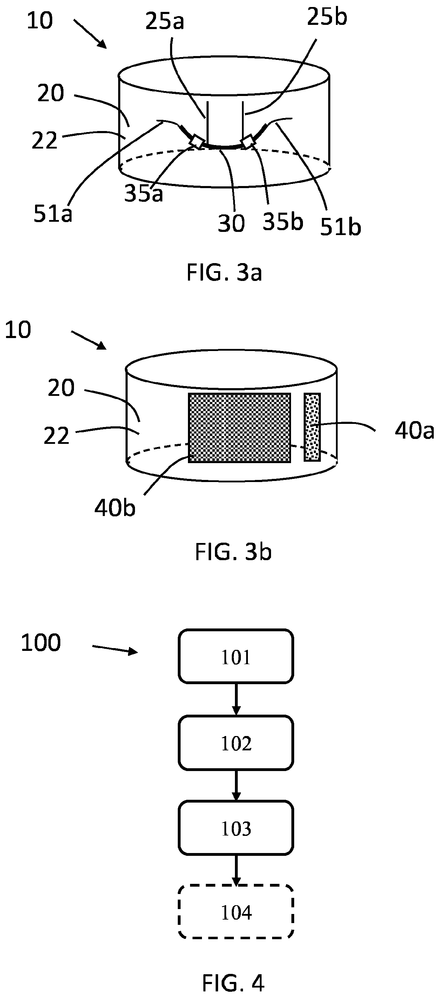

The system 10 as shown in Figs 1a to 1d may comprise a fastener 40, for fastening the band around a part of the user's body, e.g., the fastener may be a buckle, a button, a hook-and-loop fastener or any other known means. A fastener is, however, not essential for the invention to work, as is further explained in Fig. 3a.

Figs 1a to 1d, show that the system 10 may also comprise a non-stretchable part 21. The non-stretchable part 21 is less stretchable than the stretchable part 22 of the band 20, e.g., it is able to stretch less than 20%, preferably less than 10% and more preferably less than 5% of its width and/or length in unstretched position.

In Figs 1a to 1d, the non-stretchable element 30 is a ribbon, mounted on the non-stretchable part 21 of the band 20 with a connector 55 at one end and an elastic joint 51 at the other end. The elastic joint 51 is made of an elastic material, e.g., rubber, which elongates when taut.

Alternatively, the ribbon may be mounted on the stretchable part 22 of the band 20 at both ends, e.g., with a connector 55 at one end, fastening the ribbon to the stretchable part 22 and an elastic joint 51 at the other end, wherein the elastic joint 51 is also fastened to the stretchable part 22. Other solutions are also possible, wherein one end of the non-stretchable element 30 is connected to the non-stretchable part 21 and the other one is connected to the stretchable part 22.

Fig. 1a shows the system 10 in an untaut position, where no tensile force is exerted to the system.

After a sufficient tensile force is exerted to the system, the ribbon is pulled straight as shown in Fig. 1b. The elastic joint 51, permits stretching of the band 20 even when ribbon is taut, i.e., the stretching of the band is not restricted up to the ribbon's length.

The relative position of the first marker 25 located on the stretchable part 22 of the band 20 and the second marker 35 located on the non-stretchable element 30 indicates an amount of stretch of the stretchable part 22. As the stretchable part 22 of the band 20 stretches, its length increases and the position of the first marker 25 changes with respect to the second marker 35. In an initial position, e.g., the stretchable part 22 being in a position as shown in Fig. 1b, where the stretchable part is stretched enough that the ribbon is pulled straight, there is a distance d, between the first marker 25 and the second marker 35. As the stretch increases, the first marker 25 moves closer to the second marker 35 and distance d between the first marker 25 and the second marker 35 decreases. At a point where the distance d becomes zero, the first 25 and second markers 35 coincide and this is the point where the amount of stretch is a desired one.

The first 25 and second 35 markers are shown in the Figures to have a relatively small size, but this is depicted for illustration purposes only. One of the first 25 and the second 35 marker may have larger dimensions from the other e.g., the second marker 35 may have a greater width than the first marker 25. In this example, the desired amount of stretch is not confined to a certain point where the first and second markers coincide, but to a certain range of stretch. For example, the desired amount of stretch is attained, as the first marker 25 reaches one end of the second marker 35 and as the stretch increases and the first marker 25 continues to move with respect to the second marker 35, the stretch is considered to be desired, up to the point that the first marker reaches another end of the second marker 35.

As the stretch continues, after the first marker 25 has reached the second marker 35, i.e., after the desired amount of stretch has been attained, the distance d between the first 25 and second 35 marker increases again as shown in Fig. 1d. In Fig. 1d the band 20 is shown in an overstretched position, as it has already exceeded the desired stretch and in Fig. 1b the band 20 is shown in an under-stretched position, as it has not reached the desired stretch.

The first marker 25 and second marker 35 are preconfigured to coincide, to the position where the stretchable part 22 of the band 20 has the desired stretch. The desired stretch corresponds to the desired force, i.e., the force desired by design and is required to press the sensor patch against the user, when the system 10 is worn. In an example, the desired force applied to each electrode of the sensor patch is at least 0.1 N, preferably at least 0.5 N and more preferably at least 1 N. Additionally or alternatively, in case the sensor patch has 13 electrodes, and the desired force applied to each electrode is e.g., 0.5 N, a pressing force of at least 13 x 0.5 = 6.5 N is needed. As not all the pressing force of the belt will be distributed to the electrodes, the desired force applied from the band 20 to the sensor patch should be for example doubled, thus it should at least be 13 N. In one non-limiting example, the desired force is a force fulfilling the following requirements: i. it comprises a vector vertical to the skin of the user, which presses the sensor patch on the user, so that the at least one electrode of the sensor patch is able to take the measurements as intended, ii. the sensor patch remains positioned on the user, even when the user changes position or moves and iii. it does not create a prolonged discomfort to the user.

The system 10 may also be considered to work as a scale. For example, by observing the relative position of the first 25 and second 35 markers, e.g., distance d in Figs 1b and 1d, the user can estimate how much extra/less stretch is needed to reach the desired amount of stretch.

First 25 and second 35 markers may be printed, sewn or drawn. Additionally, or alternatively instead of being visually perceived by the user, they may also be markers being perceived by the sense of touch, e.g. being protrusions, or having unique tactile properties. Additionally, or alternatively, the user or a third party may also be informed over the relative position of first and second markers using audible guidance. The latter two exemplary solutions would be especially helpful for visually impaired users.

The system 10 may have two or more first markers 25 on the band 20 and two or more second markers 35 on the non-stretchable element 30. In such an example, a first marker 25 and a corresponding second marker 35 make one pair of first 25 and second 35 markers, and the further first and second markers make further corresponding pairs of first and second markers. A first marker 25 and a corresponding second marker 35 make one pair of first and second markers, if they coincide in the position where the band 20 has a desired amount of stretch.

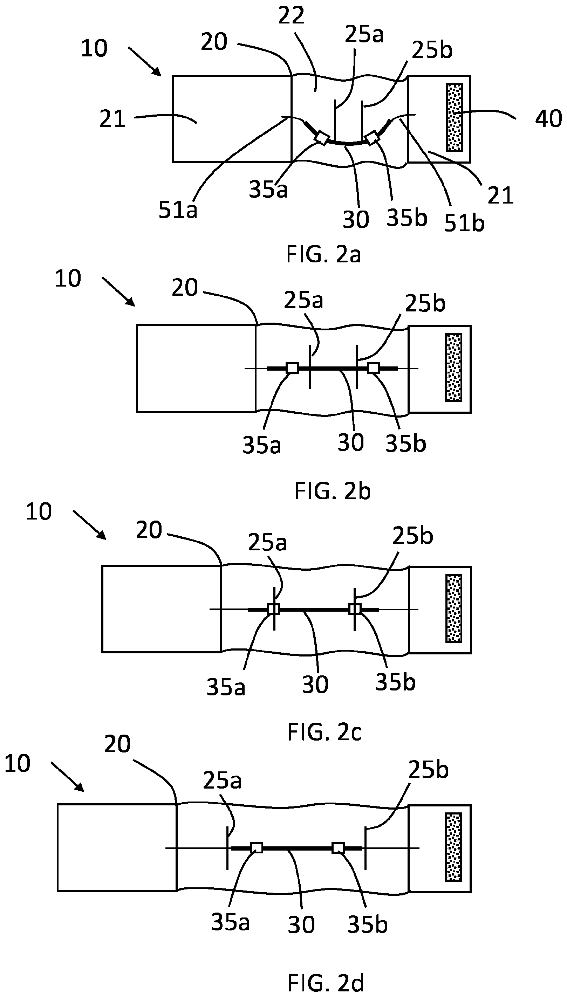

Figs. 2a to 2d show another example of a system 10. Similarly to the system 10 shown in Figs 1a to 1d, the system 10 comprises a band 20, having a stretchable part 22 and a non-stretchable part 21. It also comprises a fastener 40. The non-stretchable element 30 is a ribbon. The non-stretchable element 30 in Figs 2a to 2d has two second markers 35a and 35b, while the stretchable part 22 of the band 20 has two first markers 25a and 25b.

Similarly to the example shown in Figs 1a to 1d, the non-stretchable element 30 is mounted on the non-stretchable part 21, but it may also be mounted on the stretchable part 22. **In** Figs 2a to 2d, the non-stretchable element 30 is mounted on the band with elastic joints 51a and 51b at both of its ends.

Fig. 2a shows the system 10 in an untaut position, where no tensile forces are exerted to the system. Fig. 2b shows the system 10 under-stretched but being stretched enough that the ribbon and the elastic joints 51a and 52b are pulled straight. Fig. 2c shows the system 10 where the stretchable part 22 is under a desired stretch, as the first markers 25a and 25b coincide with the second markers 35a and 35b. Fig. 2d shows the system 10 where the stretchable part 22 is overstretched.

In the example shown in Figs 2a to 2d, the first markers 25a and 25b are symmetrical about an axis extending along a width of the band and the second markers 35a and 35b are symmetrical about the same axis. The axis may for example be chosen to pass through the middle, or other points of the non-stretchable element 30. Thus, it is ensured that the first 25 and the second 35 markers are symmetrically aligned. The user may consult the relevant position of either pair of markers, i.e., either first marker 25a compared to second marker 35a, or first marker 25b compared to second marker 35b, to check the stretch of the stretchable part 22.

Figs 1a to 2d show a band 20 having edges being configured to be fastened together, using a fastener 40. As already indicated a fastener 40 may not be used. Fig. 3a shows such an example, where the band 20 may form a closed loop e.g., may be tubular shaped, without having edges being configured to be fastened together. Such a band may be worn e.g., by pulling it though the legs or head, so that it can be worn around the torso, or around one of the extremities of the human body.

In case the band 20 comprises a fastener 40, the band 20 may have only one size, e.g., a large size. The band 20 is worn around a party of the body of the user, e.g., the belly and using the fastener 40 the amount of stretch can be adjusted. On the other hand, in case the band 20 does not comprise a fastener 40, as shown in Fig. 3a, more than one sizes of the band 20 may be available, depending on the size of the user to wear the band. The user can wear the band 20 and if it is indicated by the system that the amount of stretch is not the desired one, the user may wear another band 20 having different size, e.g., diameter, the size of this another band 20 indicating that its stretch is the desired one. In another example, more than one sizes of a band 20 without a fastener 40 may be needed for the same pregnant user for taking eCTG measurements. As pregnancy progresses, the size of the user's belly increases, thus a larger band 20 without a fastener will be probably needed to maintain a desired amount of stretch of the band holding the sensor patch.

Figs 3a and 3b show the band 20 to only comprise a stretchable part 22, nevertheless, it may also comprise a non-stretchable part. Furthermore, the solution shown in these Figures for indicating a desired stretch of the stretchable part 22 is similar to the one described in Figs 2a to 2d. Nevertheless, solutions described in all Figs 1a to 2d can be implemented with a tubular band, or any other band wherein its edges are not configured to be fastened together.

Fig. 3b shows that a fastener 40 can also be used in case of a tubular band 20, permitting the user to adjust the stretch of the stretchable part 22 of the band 20.

The examples shown in Figs 1a to 3a show a system 10 having one non-stretchable element 30. Nevertheless, more than one non-stretchable elements can be used simultaneously in the same system 10, e.g., one or more ribbons.

Fig. 4 shows a flowchart of a method for positioning a sensor patch on a user according to the invention. In step 101, the sensor patch is positioned on the user. The sensor patch may be positioned on any part of the body of the user according to the measurements to be taken, e.g., a sensor patch designed to monitor eCTG should be placed at the belly of the user. In step 102 the user places a system 10 as already described herein on the sensor patch. Thus, the system 10 provides the force which is necessary for the patch to remain positioned on the user, even when the user changes position, or moves. In an example, the sensor patch is configured to be fastened to the system 10, e.g. the sensor patch is configured to be sewn or hooked on the band 20.

After positioning the sensor patch and the system 10 on the user, in step 103, the user and/or a third party is able to monitor the amount of stretch of the stretchable part of the band indicated by the relative position of the first and second marker as already explained herein. In case the system 10 comprises a fastener 40, in an optional step 104, the user and/or a third party, can also adjust the amount of stretch of the stretchable part 22 of the band 20, by securing the fastener 40 in a desired position on the band 20. For example, if the band 20 is under-stretched, the user can increase the stretch of the band 20 up to the point where the relevant position of the first 25 and second 35 markers indicates a desired stretch. Similarly, in case the band 20 is overstretched, the user can decrease the stretch of the band 20 to the point where the relevant position of the first 25 and second 35 markers indicates a desired stretch.

In summary, a system 10 for positioning a sensor patch on a user is provided, the system comprising a band 20 for creating a pressing force to the sensor patch when the band is stretched, the band comprising a stretchable part 22, and a first marker 25 located on the stretchable part, and a non-stretchable element 30, having a second marker 35, wherein the non-stretchable element 30 is a ribbon, mounted on the band 20 with a connector 55 or an elastic joint 51b at one end and an elastic joint 51, 51a at the other end, the ribbon even when taut, permitting the stretching of the band, and wherein a relative position of the first and second marker indicates an amount of stretch of the stretchable part. The user guided by the relative position of the first 25 and second 35 markers may take actions for the stretchable part 22 of the band to reach a desired stretch, which corresponds to a desired pressing force to the sensor patch. A corresponding method 100 for positioning a sensor patch on a user is also provided.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. The invention may be implemented by means of hardware comprising several distinct elements, and/or by means of a suitably programmed processor. In the device claim enumerating several means, several of these means may be embodied by one and the same item of hardware. Measures recited in mutually different dependent claims may advantageously be used in combination.

## Claims

1. A system (10) for positioning a sensor patch on a user, the system comprising:
a band (20) for creating a pressing force to the sensor patch when the band is stretched, the band comprising a stretchable part (22), and a first marker (25) located on the stretchable part, and
a non-stretchable element (30) having a second marker (35), wherein:
the non-stretchable element (30) is a ribbon, mounted on the band (20) with a connector (55) or an elastic joint (51b) at one end and an elastic joint (51, 51a) at the other end, the ribbon even when taut, permitting the stretching of the band, and wherein:
a relative position of the first and second marker indicates an amount of stretch of the stretchable part.

2. The system of claim 1, wherein the ribbon is irreversibly mounted on the band.

3. The system of claims 1 or 2, wherein the ribbon is configured to convey a first tension force, which is less than a second tension force conveyed by the band.

4. The system of claims 1 to 3, wherein:
the stretchable part (22) comprises at least two first markers (25a, 25b), which are symmetrical about an axis extending along a width of the band (20), and
the non-stretchable element (30) is mounted on the band with elastic joints (51a, 51b) at both of its ends, wherein the non-stretchable element comprises at least two second markers (35a, 35b), which are symmetrical about the same axis.

5. The system of claims 1 to 4, wherein the band (20) comprises a fastener (40) for adjusting an amount of stretch of the stretchable part (22).

6. The system of claims 1 to 5, wherein the band (20) comprises a non-stretchable part (21), wherein the non-stretchable part is less stretchable than the stretchable part (22) and wherein at least one of the non-stretchable element (30) and the fastener (40) are mounted on the non-stretchable part.

7. A method (100) for positioning a sensor patch on a user, the method comprising:
positioning (101) the sensor patch on the user,
positioning (102) on the user a system (10) according to any of claims 1 to 6, and
monitoring (103) the amount of stretch of the stretchable part of the band indicated by the relative position of the first marker (25) and the second marker (35).

8. The method of claim 7, the method comprising:
positioning (102) on the user a system according to claim 5, and
adjusting (104) the amount of stretch of the stretchable part of the band, by securing the fastener in a desired position on the band.

## Patentansprüche

1. System (10) zum Positionieren eines Sensorpflasters auf einem Benutzer, wobei das System Folgendes umfasst:
ein Band (20), um eine Druckkraft auf das Sensorpflaster auszuüben, wenn das Band gedehnt wird, wobei das Band einen dehnbaren Teil (22) und einen ersten Marker (25) umfasst, der sich auf dem dehnbaren Teil befindet, und
ein nicht dehnbares Element (30), das eine zweite Markierung (35) aufweist, wobei:
das nicht dehnbare Element (30) ein Band ist, das an einem Ende mit einem Verbinder (55) oder einem elastischen Gelenk (51b) und am anderen Ende mit einem elastischen Gelenk (51, 51a) auf dem Band (20) montiert ist, wobei das Band auch im gespannten Zustand das Dehnen des Bandes (20) zulässt, und wobei:
eine relative Position der ersten und zweiten Markierung das Ausmaß der Dehnung des dehnbaren Teils anzeigt.

2. System nach Anspruch 1, wobei das Band irreversibel auf dem Band montiert ist.

3. System nach Anspruch 1 oder 2, wobei das Band so konfiguriert ist, dass es eine erste Zugkraft überträgt, die geringer ist als eine zweite Zugkraft, die vom Band (20) übertragen wird.

4. System nach Ansprüchen 1 bis 3, wobei:
der dehnbare Teil (22) mindestens zwei erste Markierungen (25a, 25b) umfasst, die symmetrisch zu einer Achse angeordnet sind, die sich entlang einer Breite des Bandes (20) erstreckt, und
das nicht dehnbare Element (30) mit elastischen Gelenken (51a, 51b) an beiden seiner Enden auf dem Band montiert ist, wobei das nicht dehnbare Element mindestens zwei zweite Markierungen (35a, 35b) umfasst, die symmetrisch zu derselben Achse angeordnet sind.

5. System nach Ansprüchen 1 bis 4, wobei das Band (20) einen Verschluss (40) zum Einstellen des Dehnungsausmaßes des dehnbaren Teils (22) umfasst.

6. System nach den Ansprüchen 1 bis 5, wobei das Band (20) einen nicht dehnbaren Teil (21) umfasst, wobei der nicht dehnbare Teil weniger dehnbar ist als der dehnbare Teil (22) und wobei mindestens eines von dem nicht dehnbaren Element (30) und dem Verschluss (40) auf dem nicht dehnbaren Teil montiert ist.

7. Verfahren (100) zum Positionieren eines Sensorpflasters auf einem Benutzer, wobei das Verfahren Folgendes umfasst:
Positionieren (101) des Sensorpflasters auf dem Benutzer,
Positionieren (102) eines Systems (10) nach einem der Ansprüche 1 bis 6 auf dem Benutzer, und
Überwachen (103) des durch die relative Position der ersten Markierung (25) und der zweiten Markierung (35) angezeigten Dehnungsausmaßes des dehnbaren Teils des Bandes.

8. Verfahren nach Anspruch 7, umfassend:
Positionieren (102) eines Systems nach Anspruch 5 auf dem Benutzer, und
Einstellen (104) des Dehnungsausmaßes des dehnbaren Teils des Bandes durch Sichern des Verschlusses in einer gewünschten Position auf dem Band.

## Revendications

1. Système (10) de positionnement d'un patch de capteur sur un utilisateur, le système comprenant :
une bande (20) destinée à créer une force de pression sur le patch de capteur lorsque la bande est étirée, la bande comprenant une partie étirable (22) et un premier marqueur (25) situé sur la partie étirable, et
un élément non étirable (30) présentant un second marqueur (35), dans lequel :
l'élément non étirable (30) est un ruban, monté sur la bande (20) avec un connecteur (55) ou un joint élastique (51b) à une première extrémité et un joint élastique (51, 51a) à l'autre extrémité, le ruban même lorsqu'il est tendu, permettant l'étirement de la bande, et dans lequel :
une position relative du premier et du second marqueur indique une quantité d'étirement de la partie étirable.

2. Système selon la revendication 1, dans lequel le ruban est monté de manière irréversible sur la bande.

3. Système selon les revendications 1 ou 2, dans lequel le ruban est configuré pour transmettre une première force de tension, qui est inférieure à une seconde force de tension transmise par la bande.

4. Système selon les revendications 1 à 3, dans lequel :
la partie étirable (22) comprend au moins deux premiers marqueurs (25a, 25b), qui sont symétriques par rapport à un axe s'étendant sur une largeur de la bande (20), et
l'élément non étirable (30) est monté sur la bande avec des joints élastiques (51a, 51b) à ses deux extrémités, dans lequel l'élément non étirable comprend au moins deux seconds marqueurs (35a, 35b), qui sont symétriques autour du même axe.

5. Système selon les revendications 1 à 4, dans lequel la bande (20) comprend une attache (40) permettant d'ajuster une quantité d'étirement de la partie étirable (22).

6. Système selon les revendications 1 à 5, dans lequel la bande (20) comprend une partie non étirable (21), dans lequel la partie non étirable est moins étirable que la partie étirable (22), et dans lequel au moins un des éléments non étirables (30) et la fixation (40) sont montés sur la partie non étirable.

7. Procédé (100) de positionnement d'un patch de capteur sur un utilisateur, le procédé comprenant les étapes consistant à :
positionner (101) le patch de capteur sur l'utilisateur,
positionner (102) sur l'utilisateur un système (10) selon l'une quelconque des revendications 1 à 6, et
surveiller (103) la quantité d'étirement de la partie étirable de la bande indiquée par la position relative du premier marqueur (25) et du second marqueur (35).

8. Procédé selon la revendication 7, le procédé comprenant les étapes consistant à :
positionner (102) sur l'utilisateur un système selon la revendication 5, et
ajuster (104) la quantité d'étirement de la partie étirable de la bande, en fixant l'attache dans une position souhaitée sur la bande.
